# EUROPEAN PATENT APPLICATION

(11) **EP 1 930 729 A1**
(43) Date of publication of application: **11.06.2008**
(21) Application number: 06025051.1
(22) Date of filing: 04.12.2006
(51) Int. Cl.: G01N 33/68, G01N 33/543

(54) **Method for in vivo and in vitro testing for a pharmaceutical drug induced immediate type hypersensitivity reactions**

(71) Applicant: Medizinische Universität Wien, 1090 Wien (AT)
(72) Inventor: Jensen-Jarolim, Erika, Prof. Dr., 1210 Wien (AT); Riemer, Angelika, Dr., 1080 Wien (AT); Kinaciyan, Tamar, Prof. Dr., 1180 Wien (AT)
(74) Representative: Kador & Partner

(57) **Abstract**

The present invention provides a method of testing for type I allergy towards a pharmaceutical drug using a drug-carrier conjugate, which reduces false positive or negative results and avoids undesired systemic reaction in the patient.

## Description

The present invention relates to a method of testing a pharmaceutical drug for type I allergy response and further to a drug-carrier conjugate used in such method.

Allergic reactions towards drugs are increasingly observed in industrialized countries, although exact epidemiological studies are missing. Drug hypersensitivity is a serious problem, because it may cause life threatening reactions in patients.

It is known that 10-12% of outpatients suffer from an adverse drug reaction (ADR), which in 2 to 5% of cases then require inpatient treatment due to high morbidity. A meta-analysis of 39 prospective studies from US hospitals showed that the incidence of ADR in inpatients was 15.1%. 6.7% of them suffered from severe ADR and in about 0.32% of these cases the outcome was fatal (Lazarou et al., Jama 1998; 279, 1200-12005; and Wolkentstein et al., Drug Saf 1995, 13, 56-68). This is equivalent to about 100,000 deaths per year in the USA.

The skin is the most frequently affected organ and also acts as an important indicator for internal organ involvement. A diagnosis of a cutaneous drug reaction is made in about 3% of all dermatological patients. These reactions may be of allergic (immunologic), pseudo-allergic or toxic nature.

In reactions towards drugs it is essential to differentiate between allergic type I reactions and non-immunologic, pseudoallergic reactions. Both may manifest themselves clinically as pruritus, flush, urticaria, angioedema and/or dyspnea, asthma and/or hypotension and syncope; so called systemic reactions. These systemic reactions are more pronounced and life threatening (anaphylaxis) in case of true type I allergic reactions.

Type I allergic reactions are associated with the development of allergen-specific IgE antibodies which have first to be synthesized in the patient during a phase of sensitization. The formed allergen-specific IgE sensitizes effector cells in the skin and at mucosal sites. Upon consecutive allergen contact mediator release from mast cells and basophils can be induced. Cross-linking of at least two receptor-bound IgE antibodies is a critical precondition for successful triggering and release of mediators like histamine inducing allergic symptoms (Schöll et al., J. Immunol 2005; 175, 6645-6650).

Type I allergy is commonly elicited by pharmaceutical drugs such as antibiotics, analgetics, non-steroidal anti-inflammatory drugs (NSAID), radio contrast media, cardiovascular drugs, narcotics, muscle relaxants, heparin, low molecular weight heparins, local anesthetics, colloidal expanders, drugs on protein basis, insulin, protamine, recombinant GM-CSF, cytokines, biologicals etc..

However, the diagnosis of drug related type I allergic reactions is currently not satisfying, because there are no reliable and standardized test substances, neither for in vivo nor for in vitro test methods available for drug allergy.

In the in vitro test methods specific immunoglobulins can only rarely be found in the patient's serum. The reason is the low coupling efficacy of drugs (having usually a low molecular weight) to solid phases used for immunological assays.

Furthermore, clinical diagnoses are performed with unmodified drugs (skin prick tests, intradermal tests and, if needed, drug provocation tests) by only a few clinics with special expertise, as the tests available are cumbersome for the patient, time consuming and expensive due to the drug provocation testing as a last step and today's golden standard, that is performed under continuous cardiac monitoring on a ward in hospitalized patients.

Intradermal tests are associated with the risk of systemic reactions. In addition, they can produce false positive test results in non-sensitized persons due to non-specific mast cell degranulation, e.g. in case of opiates, codein or radio contrast media.

On the other hand, both skin prick and intradermal tests often remain negative in drug sensitized patients.

Thus, one object of the present invention is to provide a reliable and standardized test substance useful in vivo and in vitro diagnostic tests for type I allergic reactions triggered by a pharmaceutical drug, and to avoid false positive or negative test results as well as to decrease the risk of systemic reactions in the patient during skin testing, especially in intradermal tests.

A further objective of the present invention in this context is to find highly effective drug-carrier conjugates which in type I allergy enable drug-antigen presentation to drug specicific antibodies a) found in the patient's serum (in vitro tests) and b) to cell bound drug specific antibodies localised in the patient's skin (in vivo tests) in a highly reliable and accurate manner.

In the present invention it was found that these objects can be achieved by direct coupling of the pharmaceutical drug to a pharmaceutically acceptable carrier.

Thus, the present invention provides an in vitro method of testing a pharmaceutical drug for type I allergy response, which comprises the following method steps
a. Coupling said pharmaceutical drug directly to a pharmaceutically acceptable carrier to form a polymeric drug-carrier conjugate,
b. Coating a solid phase with said drug-carrier conjugate of step (a),
c. Incubation of the coated solid phase with a test sample, subsequently
d. Detecting of immunoglobulins contained in the serum test-sample by a further labelled antibody, and
e. Determining from said detection step (d) the type I allergenic potential of said pharmaceutical drug.

In the present invention the term "pharmaceutical drug" means any substance which shows a medicinal effect in a patient, e.g. for curing, alleviating, or modifying clinical symptoms or causes. Thus, the substance is used as a medicament or in a medicament as active substance. The triggering of a type I allergic reaction in a patient is an undesired side effect of the pharmaceutical drug.

In the present invention it is shown that the coupling of a pharmaceutical drug directly to a pharmaceutically acceptable carrier leads to an effective coating to the solid phase used in immunological assays and to detectable signals of bound immunoglobulins contained in the test sample.

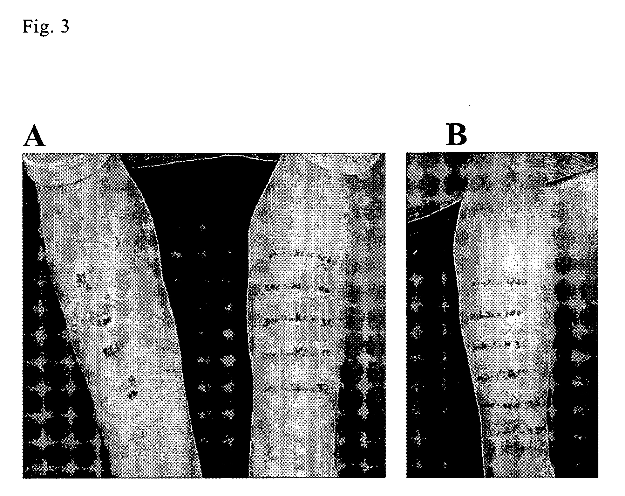

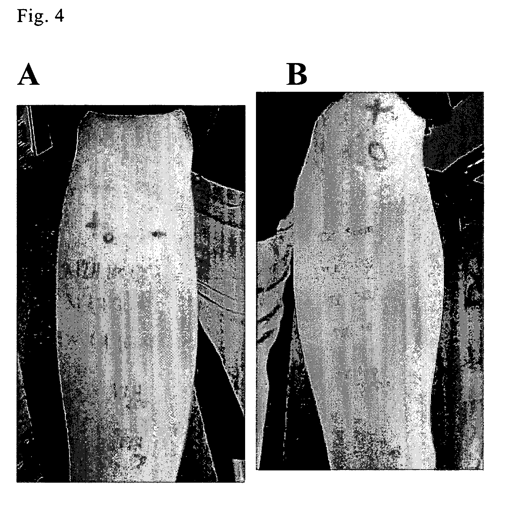

It is further demonstrated below that different liquid concentrations of the conjugated pharmaceutical drug show reliable positive skin prick test results in patients (Figure 3 and 4). Thus, the present invention reduces false negative results in the shown cases of type I allergy but also false positive result in the control cases and avoids undesired systemic reactions in the patient by putting the intradermal test aside which is performed as the next step in case of false negative skin prick test results and is not rarely associated with systemic side effects.

In the present invention the pharmaceutical drug is bound to the carrier directly, i.e. without the use of any further chemical compound, which functions as a spacer between the drug and the carrier. Thus, there is no further substance, which may mediate undesired effects during the in vitro test method or the in vivo testing in the patient. Furthermore, the production of such drug-carrier conjugate is easy, fast and does not accrue high cost.

As mentioned above, type I allergic reactions are commonly elicited by pharmaceutical drugs such as antibiotics, analgetics, non-steroidal anti-inflammatory drugs (NSAID), radio contrast media, cardiovascular drugs, narcotics, muscle relaxants, heparin, low molecular weight heparins, local anesthetics, colloidal expanders, drugs on protein basis, insulin, protamine, recombinant GM-CSF, cytokines, biologicals and their like.

Hence, the method of the present invention relates preferably to all pharmaceutical drugs which are selected from the above mentioned groups.

In the following section, specific examples of drug compounds which may be tested by the method of the present invention are listed. However, any type one allergic reaction caused by pharmaceutical drug known in the art can be tested by the method of the present invention.

List of the most important drugs eliciting type I allergy, which may be tested in the present invention:

### 1. Antibiotics:

- *Betalactams:*: penicillin G & V, amoxicillin, ampicillin, oxacillin, piperacillin
- *Monobactams:*: aztreonam
- *Carbapenems:*: imipenem,
- *Cephalosporis:*: generation I - IV: cephalexin, cefamandol, cefaclor, cefuroxim, ceftriaxon.ceftazidimm, cefepim
- *Macrolids:*: erythromycin, clarithromycin, josamycin,
- *Tetracyclin:s*: doxycyclin
- *Clindamycin*:
- *Chinolones:*: gyrase inhibitors: ofloxacin, norfloxacin, ciprofloxacin, levofloxacin
- *Aminoglycosides:*: gentamicin, tobramycin
- *Glycopeptides:*: vancomycin, teicoplanin
- *Fosfomycin*:
- *Sulfonamides:*: sulfamethoxazole, trimethoprim

### 2. Analgetics, nonsteroidal anti-inflammatory drugs (NSAID)

Acetylsalicylic acid (2-acetyloxybenzoic acid), diclofenac (2-[2-(2,6-dichlorophenyl)aminophenyl]acetic acid), paracetamol (N-(4-hydroxyphenyl)acetamide), phenylbutazone (4-butyl-1,2-diphenyl-3,5-pyrazolidinedione), mefenamic acid (2-[(2,3-dimethylphenyl)amino]benzoic acid), metamizole ((1,5-dimethyl-3-oxo-2-phenyl-pyrazol-4-yl)methylamino]methanesulfonic acid), piroxicam ((8E)-8-[hydroxy-(pyridin-2-ylamino)methylidene]-9-methyl-10,10-dioxo-10$1^{6}-thia-9-azabicyclo[4.4.0]deca-1,3,5-trien-7-one), lornoxicam ((3E)-8-chloro-3-[hydroxy-(pyridin-2-ylamino)methylidene]-4-methyl-5,5-dioxo-5$1^{6},9-dithia-4-azabicyclo[4.3.0]nona-7,10-dien-2-one), ibuprofen (2-(4-isobutylphenyl) propionic acid) or a salt thereof e.g. sodium salts of the acid components.

### 3.Heparins, low molecular weight heparins (LMWH)

- *Heparin:*: heparin-Na, heparin-Ca
- *LMWHs:*: dalteparin, enoxaparin, certoparin, nadroparin

### 4. Local anesthetics

- E*ster- compounds:*: procain, tetracain benzocain,
- *Amide-compounds:*: butanilicain, mepivacain, bupivacain, etidocain, lidocain, prilocain, articain

### 5. General anesthetics, muscle relaxants, narcotics

- *Introduction preparations:*: thiopental, propofol, midazolam, ketamine
- *Muscle relaxants:*: succinyl choline, atracurium, recuronium pancuronium, mivacurium
- *Narcotics:*: morphium, codeine, fentanyl,
(Other drugs used in general anesthesia: atropin, neostigmin, aprotinin,protamin, dextran, albumin, gelatine)

### 6. Radio contrast media

- *Ionic-monomeric:*: Na-diatrizoate
*Ionic-dimeric:* Na-ioxaglat meglumin
- *nonionic-monomeric:*: iopamidole
iohexol
- *nonionic-dimeric:*: iodixanole

### 7. Cardiovascular drugs

- *ACE-inhibitors:*: captopril, enalapril, lisinopril
- *ATI-R-antagonists:*: valsartan
- *Calcium-channel blockers:*: verapamil, nifedipine, diltiazem

It is moreover preferred that immediate type allergic reactions triggered by the above mentioned non-steroidal anti-inflammatory drugs (NSAID) is determined by the method of the present invention.

It is most preferred that said drug is diclofenac or a salt thereof, e.g. diclofenac sodium salt.

Diclofenac is one of the most commonly used analgetics and NSAIDs which often causes immediate type I allergic reactions, such as urticaria generalized urticaria and angioedema or less often more pronounced systemic reactions such as asthma and / or hypotension and occasionally severe anaphylaxis that ends lethally if not immediately treated in an adequate manner.

The drug to be applied in the method of the present invention is chemically coupled to a pharmaceutically acceptable carrier to form a drug-carrier conjugate, wherein the drug and carrier are preferably covalently bound.

In the present invention it is preferred that the drug offers a carboxyl, amine, amide, sulfhydryl, hydroxyl, aldehyde, diazonium, acylhalogenide, hydrazine, vinyl, maleimide, succinimide, and/ or hydrazine group to form the covalent bond to the carrier.

The coupling reaction between the drug and the carrier is preferable activated by N'-Ethyl-N'-3-dimethylaminopropyl)carbodiimide hydrochloride (EDAC) and (N-hydrosuccinimide) (NHS).

As pharmaceutically acceptable carrier any multivalent (multimeric) agent, which offers chemical groups easily acceptable for coupling, may be used.

However, in the present invention it is preferred that the carrier is selected from the group consisting of keyhole limpet hemocyanin, polyethylene glycol, poly(lactic acid), poly(glycolic acid); poly(lactic-co-glycolic acid).

A specifically preferred carrier is keyhole limpet hemocyanin (KLH), which is a protein from molluscs existing in two isoforms, KLH1 and KLH2. It is composed of a polypeptide chain consisting of 400 kDa subunits folded into a series of eight globular 50 kD domains. Twenty copies form a cylindrical quaternary structure. It is safe and approved by the FDA for use in humans.

The carrier may also be exemplified by polyethylene glycol. The advantage of this material is that it is recognized as tolerogen, i.e. it has no immunogenicity per se. It offers numerous coupling sites.

A further alternative for the carrier may also be exemplified by poly(lactic acid) (PLA), poly(glycolic acid) (PGA), and/or poly(lactic-co-glycolic acid) (PLGA). These substances are also approved for use in humans. They are biodegradable and biocompatible and no immunogenicity against them is observed. Being polymers, they again offer a lot of positions for chemical coupling of drugs.

The coupling ratio between the drug and the carrier is analysed by analytic methods like mass spectroscopy. In the present invention it is preferred that the ratio of drug to carrier is at least 10:1, more preferred at least 50: 1, even more preferred at least 150: 1, and most preferred at least 300:1 based on the molecular basis.

For example the free carboxyl group of diclofenac is used to couple it to the free amino groups of approximately 300 available lysins per molecule of KLH.

For in vitro tests, in contrast to un-conjugated diclofenac, the generated drug-carrier conjugate is suitable to coat the solid phase of common immunological assays effectively and thus the drug is an optimal candidate for binding immunoglobulins contained in a serum sample.

In the present invention it is preferred that the solid phase is nitrocellulose, polystyrene or a comparable plastic surface for cell culture. It is more preferred that the solid phase is a well of a so-called ELISA plate which is commercially available. However, any material known in the art may also be used.

Furthermore, for coating the solid phase it is preferred that the drug-carrier conjugate is used in a solution, wherein the drug-carrier conjugate is diluted in a suitable buffer, such as a carbonate/bicarbonate buffer, having a pH-value of 8.5, more preferably of 9.0, most preferably of 9.6.

The used concentration of drug-carrier conjugate is preferably at least 0.1 microgram/ml, more preferably at least 0.5 microgram/ml, even more preferably at least 1 microgram/ml, more preferably at least 2 microgram/ml, most preferably at least 5 microgram/ml of the solution. The upper limit of the used concentration is 20.0 microgram/ml.

The solution containing the drug-carrier conjugate is incubated with the solid phase for at least 2 hours, preferably 10 hours, more preferably 12 hours, most preferably 18 hours.

After coating of the solid phase the coated solid phase is incubated with the tested sample for preferably 2 to 20 hours, more preferably 10 hours, even more preferably 12 hours, most preferably 18 hours.

The sample is derived from patients, who have reported an immediate type hypersensitivity reaction to a drug, i.e. to the drug used in the drug-carrier conjugate. The sample is preferably a blood-serum of a patient. In case of drug hypersensitivity the sample contains immunglobulins binding to the drug-carrier conjugate.

In the present invention it is preferred that the immunglobulins are IgE - antibodies related to type I allergic reaction but testing is possible for any other immunoglobulin classes e.g. IgG immunoglobulins.

In a further method step of the present invention, the immunoglobulin-drug-carrier conjugate complex is detected with a labelled antibody. The labelled antibody is preferably a peroxidase-coupled antibody, or a biotin labelled antibody, or an alkaline phosphatase labelled antibody, or an antibody labelled with any other compound used in ELISA detection reactions known in the art.

If a peroxidase-coupled antibody is used, subsequently a substrate, such as 2,2'-azino-bis(3-ethylbenzthiazoline-6-sulphonic acid) (ABTS) or 3,3',5,5'-Tetramethylbenzidine (TMB) is added to the solid phase. For other labelling compounds, suitable substrates are to be used. In case the sample is derived from a drug hypersensitive patient and thus contains immunoglobulins, a signal can be determined in an ELISA reader.

In a further aspect of the present invention the above described drug-carrier conjugate is used for in vivo tests in patients who have reported immediate type hypersensitivity reactions to determine the type I allergenic potential of a pharmaceutical drug, for example NSAID or any other drug listed above.

For the in vivo diagnosis the above described drug-carrier conjugate is solved in a sterile, neutral fluid in known different concentrations.

Afterward the tests containing the drug-carrier conjugate are kept in a sterile manner in the refrigerator.

The in vivo test used in the present invention is a so-called skin prick test. For said skin prick testing one drop of each concentration of these standardized, sterile test solutions are applied to the volar skin of the inner forearm of the patient after having degreased and disinfected this skin part and labelled or marked the skin for each test or each test concentration of a test substance.

The skin is then briefly pricked perpendicularly through each test drop with a special prick lancet (a separate lancet is used for each test drop) by a trained nurse or doctor without causing bleeding but allowing the soluble in a polymeric manner conjugated drug to reach the dermis (deeper part of the skin) through the minimal skin dehiscence.

The test is read after 20 minutes. As can be seen from Figure 3 and 4, all test sites where a wheal and flare reaction comparable with the wheal of the positive control has formed can be counted as positive.

In the present invention an immediate positive type reaction to the tested drug in a dose-dependent fashion can be observed, in case the patient is drug hypersensitive.

In the present invention it is preferred that the sterile, neutral fluid is a suitable buffer as e.g. 0.9% NaCl.

It is also preferred that the dilutions of the drug-carrier conjugate and the buffer have concentrations ranging from 5 microgram/ml to 550 microgram/ml of drug-carrier conjugate in the suitable buffer. For example dilutions having concentrations of 500 microgram/ml, 100 microgram/ml, 30 microgram/ml and 10 microgram/ml may be used in the present invention. In the following the effectiveness of the methods of the present invention for in vitro and in vivo testing are shown.

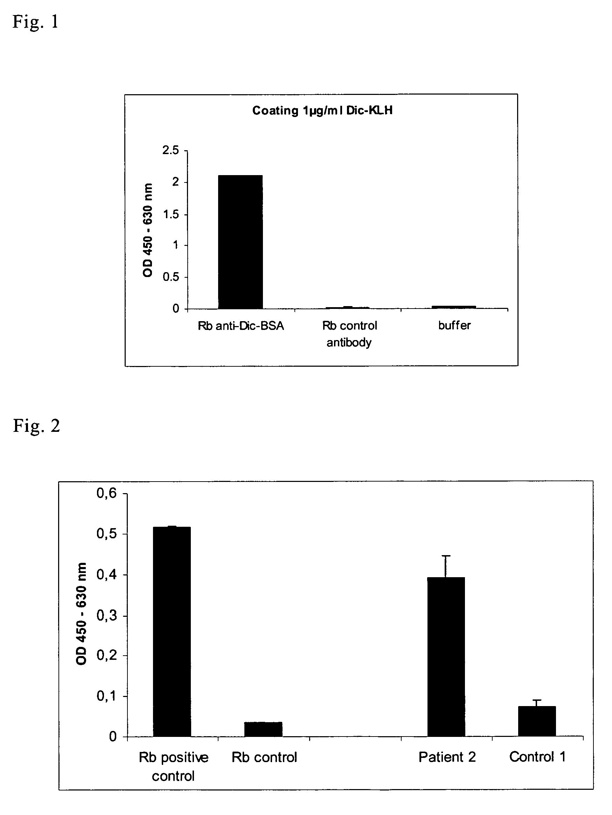

- Figure 1:: ELISA method using a drug-KLH conjugate for coating diclofenac-KLH conjugate or diclofenac alone were coated onto plates, incubated with a rabbit anti-diclofenac antibody or a control antibody, and bound antibodies were detected by a peroxidase-labeled anti-rabbit antibody. 'Buffer' shows the control incubation with the second antibody only. Y-axis: OD values (450-630 nm). The data of "dicolfenac alone" are not shown, because un-conjugated diclofenac was unable to coated the plates, and thus no antibodies of the sample were bounded.
- Figure 2:: Diclofenac-KLH conjugate was coated onto plates and the coating efficacy controlled with rabbit antibodies (Rb positive control, Rb control). Serum of patient 2 contained IgE (panel IgE) and IgG (panel IgG) towards diclofenac, whereas a serum of a control person showed no diclofenac reactivity. Bound rabbit antibodies, IgE, or IgG, respectively, were detected by peroxidase-labelled secondary antibodies. Y-axis: OD values (450-630 nm).

In the figures 3 and 4 the effectiveness of the created test substance of the present invention for in vivo testing e.g. skin prick test in 2 diclofenac allergic patients are shown.
- Figure 3:: (Patient 1), Panel A: The diclofenac-KLH conjugate was diluted in serial dilutions and skin prick tested in comparison to uncoupled diclofenac (Diclobene^{®}) (left arm) or KLH alone (right arm of patient). Note the specific and sustained reactivity with the conjugate only, which occurs in a dose-dependent manner.Panel B: Consecutive intradermal test in the same diclofenac allergic patient. Diclofenac (Diclobene^{®}) was tested intradermally on the wrist of the left arm, the positive control for the test is seen above the elbow.
- Figure 4:: (Patient 2), Panel A: The diclofenac-KLH conjugate was diluted in serial dilutions and tested in comparison to the same dilutions of KLH alone (right arm) and Panel B: to uncoupled diclofenac (Diclobene^{®}) with a higher concentration in addition to the same serial dilution as in diclofenac-KLH conjugate (left arm).

Note the specific and sustained reactivity with the conjugate only in a dose dependent manner. In this case a relatively weaker specific reactivity could be observed with diclofenac intradermally tested on the wrist (panel B). The respective positive control for the intradermal test is seen above the elbow.

### Examples

In all tests diclofenac was used as a NSAID.

### 1. In vitro method for diagnosis of type I drug allergy

Diclofenac sodium salt was coupled by its free carboxy group to the free amino groups of approximately 300 available lysins of KLH. The free amino group of KLH was activated by N-Ethyl-N'-(3-dimethylaminopropyl)carbodiimide hydrochloride (EDAC) and N-hydrosuccinimide (NHS). After coupling, the conjugate was desalted by a PD10 column. This method is described in detail in Greg T. Hermanson, Bioconjugate Techniques, Academic Press, San Diego, California, 1996.

Afterwards, the above described drug-carrier conjugate was used for coating the ELISA plate in a concentration of 1 microgramm/ml carbonate/bicarbonate coating buffer (pH 9.6). After blocking with phosphate buffered saline (PBS) containing 1% casein, the wells were incubated with a positive control rabbit anti-diclofenac antiserum, or with a rabbit antiserum against an irrelevant antigen as a negative control, both diluted 1:5000 in PBS containing 0.1% casein. After washing with PBS containing 0.05% Tween 20, bound rabbit immunoglobulin was detected with a peroxidase-coupled mouse anti-rabbit antibody. After TMB substrate addition and development, the signal intensity was determined in an ELISA reader at OD ₄₅₀₋₆₃₀ₙₘ.

As can be seen from Figure 1 the diclofenac-KLH conjugate has a high coating efficacy for diagnostic ELISAs. In contrast, diclofenac alone could not be coated.

In a further experiment, the diagnostic value of the solid phase assay was demonstrated. A patient's serum was diluted 1:4 and incubated with diclofenac-KLH coated ELISA plates overnight. After washing (as described above) bound IgE was detected by a peroxidase-labelled mouse anti-human IgE antibody (1:1000) and the colour reaction developed. For IgG detection, the patient'serum was diluted 1:100, and bound IgG was detected by a peroxidase-labelled mouse anti-human IgG antibody.

Figure 2 shows that IgE and IgG against diclofenac can be specifically detected in the serum of this patient (patient no. 2).

### 2. Allergological in vivo method for diagnosis of drug hypersensitivity

The diclofenac-KLH conjugate was applied in skin prick testing of 2 human patients (volunteers, with informed consent) who reported diclofenac hypersensitivity, and in 2 healthy controls (all volunteers, with informed consent). It could be observed that diclofenac-KLH rendered positive skin reacticity, whereas diclofenac alone, or KLH alone did not. The results show further that the extent of reactivity with diclofenac-KLH in skin prick testing was dose-dependent (500 microgram/ml, 100 microgram/ml, 30 microgram/ml and 10 microgram/ml were tested) and specific (Table 1.).

**Table 1: Prick Tests with the drug conjugate diclofenac-KLH**

| diclofenac-KLH | Patient 1 | Patient 2 | Control 1 | Control 2 |
|---|---|---|---|---|
| 500 µg/ml | 17 mm¹ | n.d.*²* | 0 mm | 0 mm. |
| 100 µg/ml | 15 mm | 13 mm | n.d. | n.d. |
| 30 µg/ml | 7 mm | 6 mm | n.d. | n.d. |
| 10 µg/ml | 0 mm. | 4 mm | n.d. | n.d. |

| | | | | |
|---|---|---|---|---|
| ¹ diameter of erythema (flare), ² n.d.: not done | | | | |

In contrast, skin prick testing with the uncoupled drug (Diclobene^{®} Ampulla) remained negative even at a concentration of 37,5 mg/ml and required intradermal testing in a second step for diagnosis. Here, diclofenac (Diclobene^{®} Ampulla) was used at a concentration of 375 microgram/ml and a volume of 20 microliter injected. Testing of two control persons without diclofenac hypersensitivity did not elicit immediate type reactivity with any of the substances.

Intradermal testing is more demanding for the patient and needs specially trained staff, because there is an increased risk of anaphylactic reactions. In accordance with the drug skin test performing guidelines a peripheral line and ready available emergency equipment are needed for drug skin testings. Thus, taken together, testing with the conjugate renders diagnosis and makes intradermal testing superfluous. Considering that the protein amounts given above refer to the whole conjugate, also the net amount of the drug is reduced significantly being in conjugated form. Furthermore, in skin prick testing the epidermis is only slightly punctured, whereas upon intradermal application volumes of above 10 microliter are necessary to elicit reactions.

As it has been demonstrated above, the direct coupling of a pharmaceutical drug to a pharmaceutically acceptable carrier leads to highly effective drug-carrier conjugates which enable type I allergy tests in vivo and in vitro in a highly reliable and accurate manner. The method reduces false positive or negative test results and decreases the risk of systemic reactions in the patient.

## Claims

1. Method of testing a pharmaceutical drug for type I allergy response comprising the steps
a.) Coupling said pharmaceutical drug directly to a pharmaceutically acceptable carrier to form a drug-carrier conjugate,
b.) Coating a solid phase with said drug-carrier conjugate of step (a),
c.) Incubation of the coated solid phase with an test-sample, subsequently
d.) Detecting of immunoglobulins contained in the test-sample by a further labelled antibody, and
e.) Determining from said detection step (d) the type I allergenic potential of said pharmaceutical drug.

2. The method according to claim 1, wherein said carrier and pharmaceutical drug are covalently coupled.

3. The method according to claims 1 or 2, wherein said pharmaceutical drug is selected from the group consisting of antibiotics, analgetics, non-steroidal anti-inflammatory drugs, radio contrast media, cardiovascular drugs, narcotics, muscle relaxants, heparin, low molecular weight heparins, local anaesthetics, colloidal expanders, drugs on protein basis, insulin, protamine, recombinant GM-CSF, cytokines and biologicals.

4. The method according to any of the preceding claims, wherein the coupling of said pharmaceutical drug to carrier is mediated by a carboxyl, amine, amide, sulfhydryl, hydroxyl, aldehyde, diazonium, acylhalogenide, hydrazine, vinyl, maleimide, succinimide, and/ or hydrazine group of said drug.

5. The method according to any of the preceding claims, wherein said pharmaceutical drug is a non-steroidal anti-inflammatory drug.

6. The method according to claim 5, wherein said pharmaceutical drug is selected from the group consisting of diclofenac acid, acetylsalicyl acid, paracetamol, phenylbutazone, propyphenazone, mefenamin acid, metamizol, piroxicam, lornoxicam, ibuprofen and acceptable derivates thereof.

7. The method according to any of the preceding claims, wherein said pharmaceutical drug is diclofenac acid or a salt thereof.

8. The method according to any of the preceding claims, wherein said carrier is a multivalent carrier.

9. The method according to any of the preceding claims, wherein said carrier is selected from the group consisting of keyhole limpet hemocyanin, polyethylene glycol, poly(lactic acid), poly(glycolic acid); poly(lactic-co-glycolic acid).

10. The method according to any of the preceding claims, wherein in said drug-carrier conjugate the ratio of drug to carrier is at least 10:1 on a molecular basis.

11. The method according to any of the preceding claims, wherein the solid phase is selected from the group consisting of nitrocellulose, polystyrene or comparable plastic surfaces for cell culture.

12. The method according to any of the preceding claims, wherein the drug-carrier conjugate is used for coating in a concentration of at least 0.1 microgram/ml.

13. The method according to any of the preceding claims, wherein the detected immunoglobulins are IgE.

14. A pharmaceutical drug carrier conjugate for the use in the method according to any of claims 1 to 12 to determine the type I allergenic potential of said pharmaceutical drug.

15. Use of a pharmaceutical drug-carrier conjugate in a test method according to any of claims 1 to 12 to determine the type I allergenic potential of said pharmaceutical drug.
